# EUROPÄISCHE PATENTSCHRIFT

(11) **EP 0 987 058 B1**
(45) Veröffentlichungstag und Bekanntmachung des Hinweises auf die Patenterteilung: **10.04.2002**
(21) Anmeldenummer: 99112026.2
(22) Anmeldetag: 22.06.1999
(51) Int. Cl.: B01J 35/02, C07C 67/055

(54) **Formkörper auf Basis von pyrogen hergestelltem Siliciumdioxid**
Shaped body on basis of pyrogenic produced silicon dioxide
Corps moulés à base de dioxyde de silicium produit par pyrogénation

(30) Priorität: 26.06.1998 DE 19828491
(43) Veröffentlichungstag der Anmeldung: 22.03.2000
(73) Patentinhaber: Degussa AG, 40474 Düsseldorf (DE)
(72) Erfinder: Tacke, Thomas, Dr., Paducah, KY 42001 (US); Schinke, Peter, 63517 Rodenbach (DE); Rotgerink, Hermanus Lansink, Dr., 63864 Glattbach (DE); Krause, Helmfried, 63517 Rodenbach (DE)

(56) Entgegenhaltungen:
- EP-A- 0 464 633
- EP-A- 0 634 208
- EP-A- 0 807 615
- DE-A- 19 538 799
- US-A- 4 510 263

## Beschreibung

Die Erfindung betrifft Formkörper auf Basis von Siliciumdioxid, ein Verfahren zu ihrer Herstellung und ihre Verwendung als Katalysator bei der Acetoxylierung von Olefinen.

Siliciumdioxide, insbesondere pyrogen hergestellte Siliciumdioxide, zeichnen sich durch extreme Feinteiligkeit und entsprechend hohe spezifische Oberfläche, sehr hohe Reinheit, sphärische Teilchenform und das Fehlen von Poren aus. Aufgrund dieser Eigenschaften finden die pyrogen hergestellten Siliciumdioxide zunehmend Interesse als Träger für Katalysatoren (D. Koth, H. Ferch, Chem. Ing. Techn. 52, 628 (1980)).

Aus DE-B 21 00 778 ist bekannt, Granulate auf Basis pyrogen hergestellter Siliciumdioxide bei der Herstellung von Vinylacetatmonomer als Katalysatorträger einzusetzen.

Aus DE-A 38 03 900 ist bekannt, zylindrische Teilchen mit gewölbten Stirnflächen auf Basis pyrogen hergestellter Siliciumdioxide bei der Herstellung von Vinylacetatmonomer als Katalysatorträger einzusetzen.

Aus DE-A 39 12 504 ist ein Verfahren zur Herstellung von Preßlingen bekannt, bei dem man Aluminiumstearat, Magnesiumstearat und/oder Graphit als Gleitmittel und Harnstoff sowie Methylcellulose als Porenbildner verwendet.

Die bekannten mit Magnesiumstearat hergestellten Preßlinge werden als Aerosil-Tabletten Nr. 350, Firma Degussa, in den Handel gebracht. Sie weisen ca. 0,4 Gew.-% Mg auf.

Aus EP 0 004 079 sind Katalysatorträger für Katalysatoren zur Synthese von Vinylacetat Monomer bekannt, die aus Strangabschnitten mit sternförmigem Querschnitt oder gerippten Strängen bestehen.

Aus EP-B 464 633 sind Katalysatoren zur Synthese von Vinylacetatmonomer, die mindestens einen Durchtrittskanal mit einem Innendurchmesser von mindestens 1 mm haben, bekannt.

Die DE-A 195 38 799 beschreibt einen wabenförmigen, überwiegend aus SiO₂ bestehenden Katalysatorträger, der nach Beispiel 1 einen Durchmesser von 25 mm, eine Stegbreite von 1 mm und einen Stegabstand von 2 mm bei einer Länge von 150 mm aufweist. Nach Belegung mit katalytisch aktiven Elementen kann der daraus resultierende Katalysator zur Herstellung von ungesättigten Estern aus Olefinen, Säuren und Sauerstoff in der Gasphase, zur Reinigung von organisch belasteter Abluft sowie zur Alkylierung von Aromaten verwendet werden.

Die WO 97/36679 beschreibt ebenfalls einen wabenförmigen Katalysatorträger, der mit SiO₂ beschichtet wurde und nach Tränkung mit Palladium und Gold sowie Aktivierung mit Kaliumacetat für die Herstellung von ungesättigten Estern eingesetzt werden kann.

Wabenförmige Katalysatoren zeichnen sich durch einen sehr niedrigen Druckverlust aus. In technischen Reaktoren, insbesondere in Rohrbündelreaktoren, gibt es jedoch bei der Verwendung von wabenförmigen Katalysatoren, insbesondere bei der Bestückung der Reaktorrohre, nicht unwesentliche Probleme: In Rohrreaktoren müßten zum Teil mehrere tausend Rohre einer technischen Anlage mit wabenförmigen Katalysatoren befüllt werden. Dabei ist darauf zu achten, daß die Wabenkörper beim Befüllen nicht abbrechen. Es ist großer Wert darauf zu legen, daß eine Randgängigkeit vermieden wird, da die Katalysatoren sonst ihre Leistung nicht erbringen können. Darüber hinaus weisen wabenförmige Katalysatormaterialien eine schlechte Wärmeleitfähigkeit in radialer Richtung auf. Das ist insbesondere in Reaktionen mit hoher Wärmetönung, wie z. b. bei Oxidationsreaktionen, von Nachteil. Aus den genannten Gründen ist zur Zeit keine technische Anwendung bekannt, in der ein Rohrbündelreaktor mit Hunderten bzw. Tausenden von Rohren mit wabenförmigen Katalysatormaterialien betrieben wird. Aus diesem Grunde werden die Reaktoren mit stückigen Formkörpern, die ebenfalls einen niedrigeren Druckabfall aufweisen, befüllt.

Aus EP-B 0 519 435 ist es bekannt, SiO₂ mittels Bindemittel zu Träger zu verpressen, die erhaltenen Träger zu glühen und die geglühten Trägerteilchen mittels Säure zu waschen, bis keine weiteren Kationen des Bindemittels mehr abgegeben werden. Desweiteren werden Trägerkatalysator, Verfahren zu seiner Herstellung, sowie seine Verwendung zur Herstellung von Vinylacetat beschrieben.

Die EP-A 0 807 615 beschreibt Preßlinge auf Basis von pyrogen hergestelltem Siliciumdioxid. Die Preßlinge können als Katalysator oder Katalysatorträger bei der Vinylacetatmonomerherstellung und der Ethylenhydratisierung eingesetzt werden. Die Preßlinge können verschiedene, zum Beispiel zylindrische, kugelförmige oder ringförmige Formen mit einem Außendurchmesser von 0,8 bis 20 mm aufweisen.

Gegenstand der Erfindung sind Formkörper auf Basis von Siliciumdioxid, welche dadurch gekennzeichnet sind, daß die Trägergeometrie durch eine Hohlzylinder-Konfiguration mit internen verstärkenden Stegen oder Speichen ausgehend von der inneren Wand des Hohlzylinders zum Zentrum der Formkörper besteht oder durch eine Vielzahl von Durchtrittskanälen charakterisiert ist, wobei der Formkörper einen Aussendurchmesser von 1 bis zu 25 mm und ein Verhältnis von Höhe zu Durchmesser von 0,2 bis 5 aufweist und als Siliciumdioxid ein pyrogen hergestelltes Siliciumdioxid eingesetzt wird. Weiterhin kann er ein Gesamtporenvolumen von 0,3 bis 1,8 ml/g sowie eine BET-Oberfläche von 5 bis 400 m²/g aufweisen.

Vorzugsweise kann der SiO₂-Anteil der erfindungsgemäßen Formkörper mehr als 99,0 Gew.-% betragen. Der Anteil an sonstigen Bestandteilen kann weniger als 0,2 Gew.-% betragen. Die erfindungsgemäßen Formkörper können daher als frei von Bindemittel bezeichnet werden. Der Abrieb kann weniger als 5 Gew.-% betragen. Die Schüttdichte kann 100 bis 700 g/l betragen.

Ein weiterer Gegenstand der Erfindung ist ein Verfahren zur Herstellung der Formkörper auf Basis von Siliciumdioxid, welches dadurch gekennzeichnet ist, daß man pyrogen hergestelltes Siliciumdioxid einem Knet- und Verformungsverfahren unterzieht, extrudiert, die Extrudate gegebenenfalls mittels einer Schneidvorrichtung auf die gewünschte Länge schneidet, bei einer Temperatur von 20 bis zu 150 °C trocknet und während eines Zeitraumes von 0,5 bis zu 10 Stunden bei einer Temperatur von 400 bis 1200 °C tempert.

In einer besonderen Ausführungsform der Erfindung kann das Siliciumdioxid ein pyrogen hergestelltes Siliciumdioxid sein. Erfindungsgemäß einsetzbares Siliciumdioxid, so auch pyrogen hergestelltes Siliciumdioxid, wird beschrieben in Ullmanns Enzyklopädie der technischen Chemie, 4. Auflage, Band 21, Seiten 451 bis 476 (1982).

Ein bevorzugter Gegenstand der Erfindung ist ein Verfahren zur Herstellung von Formkörpern auf Basis von Siliciumdioxid, welches dadurch gekennzeichnet ist, daß man pyrogen hergestelltes Siliciumdioxid mit Methylhydroxyethylcellulose, Wachs und/oder Polyethylenglycol unter Zusatz von Wasser sowie gegebenenfalls unter Zugabe einer wässrigen, ammoniakalischen Lösung homogenisiert, einem Knet- und Verformungsverfahren unterzieht, verformt und/oder extrudiert, die Formkörper gegebenenfalls mittels einer Schneidvorrichtung auf die gewünschte Länge schneidet, bei einer Temperatur von 20 bis zu 150°C trocknet und während eines Zeitraumes von 30 Minuten bis zu 10 Stunden bei einer Temperatur von 400 bis 1200°C tempert.

Zur Durchführung des erfindungsgemäßen Verfahrens können Kneter, Mischer oder Mühlen, die eine gute Homogenisierung und Verdichtung des Mischgutes ermöglichen, wie zum Beispiel Schaufel-, Wirbelschicht-, Kreisel- oder Luftstrommischer, eingesetzt werden. Insbesondere können Mischer, mit denen eine zusätzliche Verdichtung des Mischgutes möglich ist, wie zum Beispiel Pflugmischer, Kneter, Kollergänge oder Kugelmühlen, verwendet werden. Die Mischung und Knetung kann aber auch direkt im Extruder erfolgen. Die Herstellung der Formkörper kann auf Ein- oder Zweischneckenextrudern, Strangpressen als auch auf Tablettenmaschinen erfolgen. Vorzugsweise können die erfindungsgemäßen Formkörper mittels Extrudern hergestellt werden.

Vor der Formgebung kann in einer besonderen Ausführungsform der Erfindung die Mischung die folgende Zusammensetzung aufweisen:
- 50 - 90 Gew.-%: Siliciumdioxid, vorzugsweise 65-85 Gew.-%;
- 0,1 - 20 Gew.-%: Methylhydroxyethylcellulose, vorzugsweise 5-15 Gew.-%;
- 0,1 - 15 %: Wachs, vorzugsweise 5-12 Gew.-%;
- 0,1 - 15 %: Polyethylenglykol, vorzugsweise 5-10 Gew.-%

Die Formkörper können bei 400 - 1200 °C 30 Minuten bis 10 Stunden getempert werden. Durch Variation der Einsatzstoffmengen und des Preßdruckes können die Bruchfestigkeit, die spezifische Gesamtoberfläche und das Porenvolumen in einem gewissen Rahmen eingestellt werden.

Die erfindungsgemäßen Formkörper können entweder direkt als Katalysator oder als Katalysatorträger eingesetzt werden.

Für die Verwendung als Katalysatorträger können die Formkörper nach ihrer Herstellung mit einer katalytisch wirksamen Substanz in Kontakt gebracht und gegebenenfalls durch eine geeignete Nachbehandlung aktiviert werden.

Insbesondere lassen sich die Formkörper aus pyrogen hergestelltem Siliciumdioxid als Träger für den Katalysator bei der Herstellung von Vinylacetatmonomer aus Ethylen, Essigsäure und Sauerstoff verwenden.

Die erfindungsgemäßen Formkörper zeigen oder ermöglichen:
- niedrige Druckverluste
- niedrige Schüttdichten
- relativ große äußere Oberflächen pro Einheitsvolumen eines Reaktionsgefäßes
- einen verbesserten Stoff- und Wärmetransport
- eine, insbesondere im Vergleich zu bekannten wabenförmigen Katalysatoren, vergleichsweise einfache Befüllung und Entleerung von technischen RohrbündelReaktoren.

Der niedrige Druckverlust der erfindungsgemäßen Formkörper resultiert unter anderem aus deren geometrischen Abmessungen, wodurch eine extrem große freie Fläche im Querschnitt der Formkörper und/oder ein sehr hoher Lückengrad in der Katalysatorschüttung vorliegt.

Auf Basis der erfindungsgemäßen Formkörper lassen sich Katalysatoren herstellen, mit denen höhere Raum-Zeit-Ausbeuten und Selektivitäten zu erzielen sind.

Nachfolgend wird die Erfindung unter Bezugnahme auf die Zeichnungen näher erläutert:

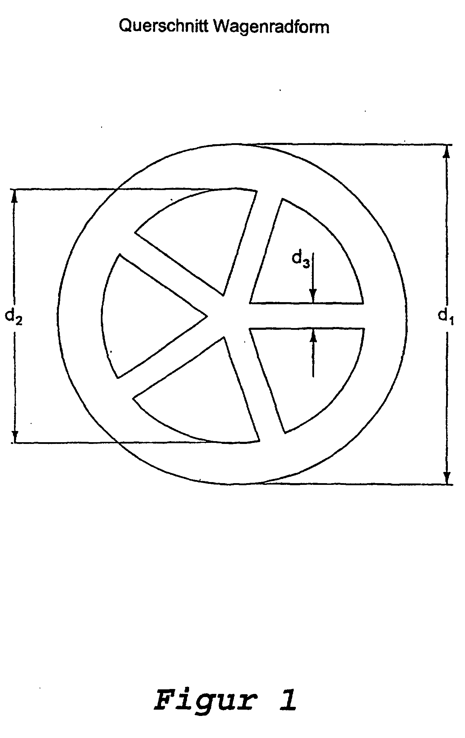

In den Zeichnungen zeigen
- Figur 1: einen Querschnitt durch einen Preßling bzw. Trägerkatalysator in Wagenradform

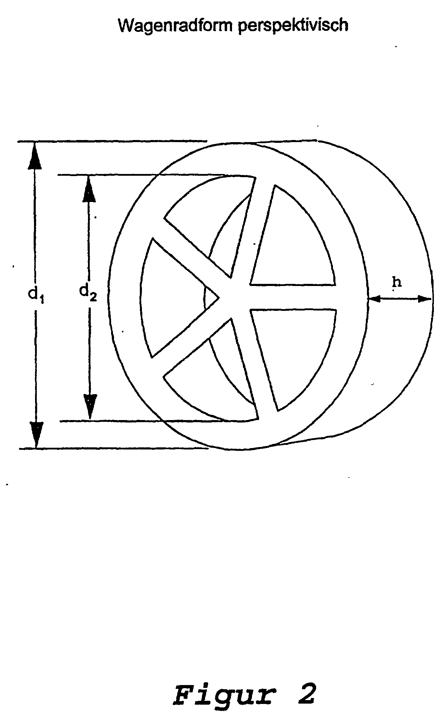

- Figur 2: eine perspektivische Darstellung der Wagenradform aus Figur 2
- Figur 3: einen Preßling bzw. Trägerkatalysator in Form von sogenannten Minilithen
- Figur 4: eine perspektivische Darstellung der Minilithe aus Figur 3.

Die Figuren betreffen erfindungsgemäße Ausführungsformen. Vorzugsweise kann der Aussendurchmesser d₁ der Wagenräder und der sogenannten Minilithe maximal 25 mm betragen, wobei das Verhältnis von Höhe h zu Aussendurchmesser (h/d₁) 0,2 bis 5 betragen kann. Der Innendurchmesser der Formkörper wird durch d₂ beschrieben. Die Wanddicke der Formkörper ((d₁ - d₂) X 0.5) kann im Bereich des 0,05 bis 0,3 fachen des Aussendurchmessers liegen. Die. Steg- bzw. Speichendicke der Formkörper wird durch d₃ beschrieben und kann im Bereich des 0,05 bis 0,3 fachen des Aussendurchmessers liegen. Die Anzahl der internen verstärkenden Stege oder Speichen oder Durchtrittskanäle kann mindestens 3 betragen.

Ein weiterer Gegenstand der Erfindung ist ein Trägerkatalysator für die Herstellung von Vinylacetatmonomer (VAM), welcher auf einem Träger (Formkörper) als katalytisch aktive Komponenten Palladium und/oder dessen Verbindungen und Alkaliverbindungen, sowie zusätzlich Gold und/oder dessen Verbindungen (System Pd/Alkali/Au) oder Cadmium und/oder dessen Verbindungen (System Pd/Alkali/Cd) oder Barium und/oder dessen Verbindungen (System Pd/Alkali/Ba) oder Palladium, Alkaliverbindungen und Mischungen aus Gold und/oder Cadmium und/oder Barium enthält, welcher dadurch gekennzeichnet ist, daß der Träger ein erfindungsgemäßer Formkörper ist.

Als Alkaliverbindungen können bevorzugt Kaliumverbindungen, wie z. B. Kaliumacetat, eingesetzt werden.

Die katalytisch aktiven Komponenten können in folgenden Systemen vorhanden sein:
Pd/Au/Alkali-Verbindungen
Pd/Cd/Alkali-Verbindungen
Pd/Ba/Alkali-Verbindungen

Die erfindungsgemäßen Trägerkatalysatoren können für die Herstellung von Vinylacetatmonomer verwendet werden. Dazu werden Ethen, Essigsäure und molekularer Sauerstoff bzw. Luft in der Gasphase, gegebenenfalls unter Zusatz von Inertgasen, bei Temperaturen zwischen 100 und 250 °C und gewöhnlichem oder erhöhtem Druck in Gegenwart des erfindungsgemäßen Trägerkatalysators zur Reaktion gebracht.

Ein derartiges Herstellverfahren ist aus den Dokumenten DE 16 68 088, US 4,048,096, EP-A 0 519 435, EP-A 0 634 208, EP-A 0 723 810, EP-A 0 634 209, EP-A 0 632 214, EP-A 0 654 301 und EP-A 0 0807 615 bekannt.

Ein weiterer Gegenstand der Erfindung ist ein Verfahren zur Herstellung des Trägerkatalysators für die Produktion von Vinylacetatmonomer durch Tränken, Aufsprühen, Aufdampfen, Tauchen oder Ausfällen der Pd-, Au, Cd-, Ba-Metallverbindungen, gegebenenfalls Reduzieren der auf dem Träger aufgebrachten reduzierbaren Metallverbindungen, gegebenenfalls Waschen zur Entfernung gegebenenfalls vorhandener Chloridanteile, Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln, in geeigneter Reihenfolge, welches dadurch gekennzeichnet ist, daß der Träger ein Formkörper gemäß dieser Erfindung ist.

Ein weiterer Gegenstand der Erfindung ist ein Verfahren zur Herstellung des Trägerkatalysators für die Produktion von Vinylacetatmonomer durch Imprägnieren des Trägers mit einer basischen Lösung und einer Gold- und Palladiumsalze enthaltenden Lösung, wobei die Imprägnierung gleichzeitig oder nacheinander, mit oder ohne Zwischentrocknung erfolgt, gegebenenfalls Waschen des Trägers zur Entfernung gegebenenfalls vorhandener Chloridanteile und Reduzieren der auf dem Träger ausgefällten unlöslichen Verbindungen vor oder nach dem Waschen, Trocknen der so erhaltenen Katalysatorvorstufe, und Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln, welches dadurch gekennzeichnet ist, daß der Träger ein Preßling gemäß Erfindung ist.

Die erfindungsgemäßen Trägerkatalysatoren können zur Herstellung ungesättigter Ester aus Olefinen, Säuren und Sauerstoff in der Gasphase eingesetzt werden.

Die erfindungsgemäßen Katalysatoren des Katalysatorsystems Pd/Alkali/Au können durch Imprägnieren der Träger mit einer basischen Lösung und einer Gold- und Palladiumsailze enthaltenden Lösung erhalten werden, wobei die Imprägnierschritte gleichzeitig oder nacheinander, mit oder ohne Zwischentrocknung erfolgen können. Anschließend wird der Träger zur Entfernung gegebenenfalls vorhandener Chloridanteile gewaschen. Vor oder nach dem Waschen können die auf dem Träger ausgefällten unlöslichen Edelmetallverbindungen reduziert werden. Die so erhaltene Katalysatorvorstufe kann getrocknet und zur Aktivierung des Katalysators mit Alkaliacetaten oder Alkaliverbindungen, die sich unter den Reaktionsbedingungen bei der Produktion von Vinylacetatmonomer ganz oder teilweise in Alkaliacetate umwandeln, imprägniert werden. Im allgemeinen können die Edelmetalle bei Pd/Au-Katalysatoren in Form einer Schale auf dem Träger vorliegen.

Bei Pd/Alkali/Ba-Katalysatoren können die Metallsalze durch Tränken, Aufsprühen, Aufdampfen, Tauchen oder Ausfällen aufgebracht werden (EP 0 519 436). Dieselben Methoden sind bei Pd/Alkali/Cd-Katalysatoren bekannt (US-PS 4,902,823, US-PS 3,393,199, US-PS 4,668,819).

Je nach Katalysatorsystem kann eine Reduktion des Trägerkatalysators vorgenommen werden.

Die Reduktion des Katalysators kann in der wäßrigen Phase oder in der Gasphase vorgenommen werden. Zur Reduktion in der wäßrigen Phase eignen sich zum Beispiel Formaldehyd oder Hydrazin. Die Reduktion in der Gasphase kann mit Wasserstoff beziehungsweise Formiergas (95 Vol.-% N₂ + 5 Vol.-% H₂), Ethen oder stickstoffverdünntem Ethen vorgenommen werden. Die Reduktion mit Wasserstoff kann bei Temperaturen zwischen 40 und 260 °C, bevorzugt zwischen 70 und 200 °C erfolgen. Die Reduktion mit Formiergas (95 Vol.-% N₂ und 5 Vol.-% H₂) kann bei Temperaturen zwischen 300 und 550 °C, bevorzugt zwischen 350 und 500 °C, erfolgen. Der Katalysator kann auch erst nach der Aktivierung mit Alkaliacetat direkt im Produktionsreaktor mit Ethen reduziert werden.

Die erfindungsgemäßen Katalysatorträger behalten unter den Reaktionsbedingungen des katalytischen Prozesses, insbesondere unter dem Einfluß von Essigsäure, vorteilhafterweise ihre mechanische Festigkeit.

Im folgenden wird die Herstellung von Trägerkatalysatoren des Systems Pd/Alkali/Au auf erfindungsgemäßen Formkörpern näher beschrieben.

Die erfindungsgemäßen Formkörper werden mit einer Palladium und Gold enthaltenden Lösung imprägniert. Gleichzeitig mit der edelmetallhaltigen Lösung oder in beliebiger Reihenfolge nacheinander werden die erfindungsgemäßen Formkörper mit einer basischen Lösung imprägniert, welche ein oder mehrere basische Verbindungen enthalten kann. Die basische Verbindung oder Verbindungen dienen zur Überführung des Palladiums und Golds in ihre Hydroxide.

Die Verbindungen in der basischen Lösung können aus Alkalihydroxiden, Alkalibicarbonaten, Alkalicarbonaten, Alkalisilikaten oder Mischungen dieser Stoffe bestehen. Bevorzugt werden Kaliumhydroxid und/oder Natriumhydroxid verwendet.

Zur Herstellung der edelmetallhaltigen Lösung können als Palladiumsalze beispielsweise Palladiumchlorid, Natriumbzw. Kaliumpalladiumchlorid oder Palladiumnitrat verwendet werden. Als Goldsalze können Gold(III)-chlorid und Tetrachlorogold(III)-säure eingesetzt werden. Vorzugsweise können Kaliumpalladiumchlorid, Natriumpalladiumchlorid und/oder Tetrachlorogoldsäure eingesetzt werden.

Das Imprägnieren der erfindungsgemäßen Formkörper mit der basischen Lösung beeinflußt die Abscheidung der Edelmetalle in dem Trägermaterial. Die basische Lösung kann entweder gleichzeitig mit der Edelmetallösung oder in beliebiger Reihenfolge mit dieser Lösung angewandt werden. Die erfindungsgemäßen Formkörper werden entweder gleichzeitig mit der basischen Lösung und mit der Edelmetallösung oder nacheinander in beliebiger Reihenfolge in Kontakt gebracht. Bei aufeinanderfolgender Imprägnierung der erfindungsgemäßen Formkörper mit den beiden Lösungen kann nach dem ersten Imprägnierschritt eine Zwischentrocknung vorgenommen werden.

Bevorzugt werden die erfindungsgemäßen Preßlinge zuerst mit der basischen Verbindung imprägniert. Die anschließende Imprägnierung mit der Palladium und Gold enthaltenden Lösung führt zur Ausfällung von Palladium und Gold in einer oberflächlichen Schale auf dem Formkörper. Die umgekehrte Reihenfolge der Imprägnierungen führt im allgemeinen zu einer mehr oder weniger homogenen Verteilung der Edelmetalle über den Querschnitt des eingesetzten Formkörpers. Bei geeigneter Verfahrensführung können jedoch auch bei umgekehrter Imprägnier-Reihenfolge Katalysatoren mit definierter Schale erhalten werden (siehe z. B. US 4,048,096). Katalysatoren mit homogener oder nahezu homogener Edelmetall-Verteilung weisen im allgemeinen eine geringere Aktivität und Selektivität auf.

Katalysatoren mit Schalendicken von unter 1 mm sind besonders geeignet. Die Schalendicke wird durch die Menge der auf den Formkörper aufgebrachten basischen Verbindung relativ zur gewünschten Menge der Edelmetalle beeinflußt. Je höher dieses Verhältnis ist, desto geringer wird die Dicke der sich ausbildenden Schale. Das für eine gewünschte Schalendicke erforderliche Mengenverhältnis von basischer Verbindung zu den Edelmetallverbindungen hängt von der Beschaffenheit des Formkörpers sowie von der gewählten basischen Verbindung und den Edelmetallverbindungen ab. Das erforderliche Mengenverhältnis wird zweckmäßigerweise durch wenige Vorversuche ermittelt. Die sich ergebende Schalendicke kann dabei in einfacher Weise durch Aufschneiden der Katalysatorteilchen ermittelt werden.

Die minimal notwendige Menge der basischen Verbindung ergibt sich aus der stöchiometrisch berechneten Menge an Hydroxidionen, die zur Überführung des Palladiums und Golds in die Hydroxide benötigt werden. Als Richtwert gilt, daß die basische Verbindung für eine Schalendicke von 0,5 mm in einem 1 bis 10-fachen stöchiometrischen Überschuß angewendet werden sollte.

Die erfindungsgemäßen Formkörper können nach dem Verfahren der Porenvolumenimprägnierung mit den basischen Verbindungen und den Edelmetallsalzen belegt werden. Wird mit Zwischentrocknung gearbeitet, wählt man die Volumina der beiden Lösungen so, daß sie jeweils etwa 90 bis 100 % der Aufnahmekapazität der eingesetzten Formkörper entsprechen. Wird auf die Zwischentrocknung verzichtet, so muß die Summe der Einzelvolumina der beiden Imprägnierlösungen der obigen Bedingung entsprechen, wobei die Einzelvolumina im Verhältnis von 1 : 9 bis 9 : 1 zueinander stehen können. Bevorzugt wird ein Volumenverhältnis von 3 : 7 bis 7 : 3, insbesondere von 1 : 1, angewendet. Als Lösungsmittel kann in beiden Fällen bevorzugt Wasser verwendet werden. Es können aber auch geeignete organische oder wäßrig-organische Lösungsmittel eingesetzt werden.

Die Umsetzung der Edelmetallsalzlösung mit der basischen Lösung zu unlöslichen Edelmetallverbindungen erfolgt langsam und ist je nach Präparationsmethode im allgemeinen erst nach 1 bis 24 Stunden abgeschlossen. Danach werden die wasserunlöslichen Edelmetallverbindungen mit Reduktionsmitteln behandelt. Es kann eine Naßreduktion zum Beispiel mit wäßrigem Hydrazinhydrat oder eine Gasphasenreduktion mit Wasserstoff, Ethen, Formiergas oder auch Methanoldämpfen vorgenommen werden. Die Reduktion kann bei Normaltemperatur oder erhöhter Temperatur und bei Normaldruck oder erhöhtem Druck, gegebenenfalls auch unter Zugabe von Inertgasen, erfolgen.

Vor und/oder nach der Reduktion der Edelmetallverbindungen wird das auf dem Formkörper gegebenenfalls vorhandene Chlorid durch eine gründiche Waschung entfernt. Nach der Waschung sollte der Formkörper weniger als 500, bevorzugt weniger als 200 ppm, Chlorid enthalten.

Der nach der Reduktion als Katalysatorvorstufe erhaltene Formkörper wird getrocknet und abschließend mit Alkaliacetaten oder Alkaliverbindungen, die sich unter den Reaktionsbedingungen bei der Produktion von Vinylacetatmonomer ganz oder teilweise in Alkaliacetate umwandeln, imprägniert. Vorzugsweise wird mit Kaliumacetat imprägniert. Hierbei wird wieder bevorzugt die Porenvolumenimprägnierung angewendet. Das heißt: Die benötigte Menge an Kaliumacetat wird in einem Lösungsmittel, vorzugsweise Wasser, dessen Volumen etwa der Aufnahmekapazität der vorgelegten Formkörper für das gewählte Lösungsmittel entspricht, gelöst. Dieses Volumen ist etwa gleich dem Gesamtporenvolumen der Formkörper.

Der fertig belegte Formkörper wird anschießend bis auf eine Restfeuchte von weniger als 2 % getrocknet. Die Trocknung kann an Luft, gegebenenfalls auch unter Stickstoff als Inertgas, erfolgen.

Die Herstellung von Trägerkatalysatoren der Systeme Pd/Alkali/Cd bzw. Pd/Alkali/Ba auf erfindungsgemäßen Formkörpern erfolgt nach den oben zitierten Patentschriften auf bekannte Weise.

Für die Synthese von Vinylacetatmonomer ist es zweckmäßig, den Formkörper mit 0,2 bis 4, vorzugsweise 0,3 bis 3 Gew.-% Palladium, 0,1 bis 2, vorzugsweise 0,15 bis 1,5 Gew.-% Gold und 1 bis 10, vorzugsweise 1,5 bis 9 Gew.-% Kaliumacetat, jeweils bezogen auf das Gewicht des eingesetzten Formkörpers, zu belegen. Diese Angaben gelten für das System Pd/Alkali/Au. Im Fall von Formkörpern mit einer Schüttdichte von 500 g/l entsprechen diese Konzentrationsangaben volumenbezogenen Konzentrationen von 1,0 bis 20 g/l Palladium, 0,5 bis 10 g/l Gold und 5 bis 50 g/l Kaliumacetat. Zur Anfertigung der Imprägnierlösungen werden die entsprechenden Mengen der Palladium- und Goldverbindungen in einem Volumen Wasser, welches etwa 90 bis 100 % der Wasseraufnahmekapazität des vorgelegten Formkörpers entspricht, gelöst. Ebenso wird bei der Anfertigung der basischen Lösung verfahren.

Der Cadmium-Gehalt der Pd/Alkali/Cd-Katalysatoren (fertig belegte Formkörper) kann im allgemeinen 0,1 bis 2,5 Gew.-%, vorzugsweise 0,4 bis 2,0 Gew.-%, betragen.

Der Barium-Gehalt der Pd/Alkali/Ba-Katalysatoren (fertig belegte Formkörper) kann im allgemeinen 0,1 bis 2,0 Gew.-%, vorzugsweise 0,2 bis 1,8 Gew.-%, betragen.

Der Palladium-Gehalt der Pd/Alkali/Cd, beziehungsweise Pd/Alkali/Ba-Katalysatoren (fertig belegte Formkörper) kann 0,2 bis 4 Gew.-%, bevorzugt 0,3 bis 3 Gew.-% Palladium betragen.

Der Kaliumacetat-Gehalt der Pd/Alkali/Cd- bzw. Pd/Alkali/Ba-Katalysatoren (fertig belegte Formkörper) kann im allgemeinen 1 bis 10 Gew.-%, vorzugsweise 1,5 bis 9 Gew.-%, betragen.

Als pyrogen hergestelltes Siliciumdioxid können Siliciumdioxide mit den folgenden physikalisch-chemischen Kenndaten, auch bekannt unter dem Namen Aerosil® der Firma Degussa, eingesetzt werden:

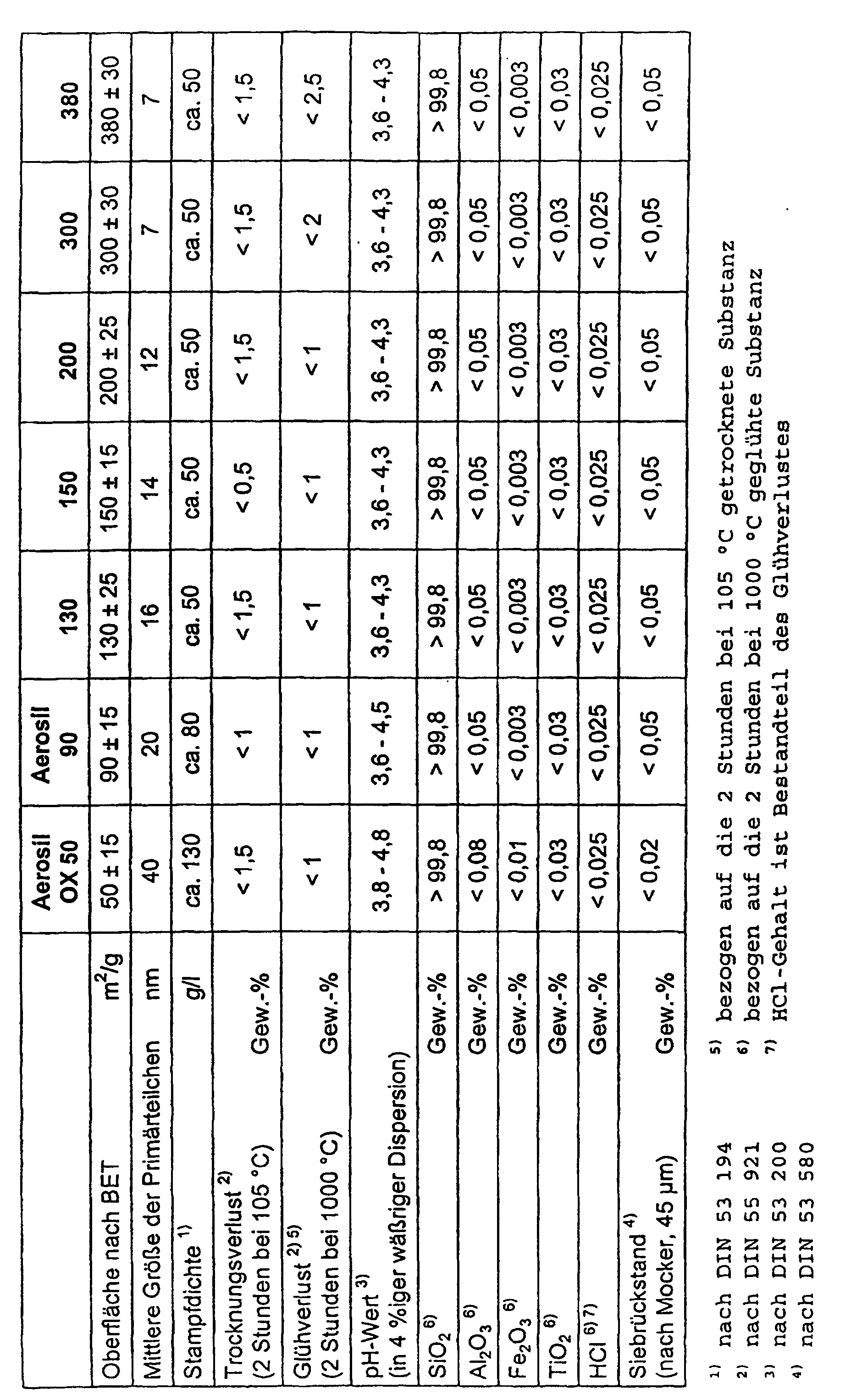

Zur Herstellung von AEROSIL® wird in eine Knallgasflamme aus Wasserstoff und Luft eine flüchtige Siliciumverbindung eingedüst. In den meisten Fällen verwendet man Siliciumtetrachlorid. Diese Substanz hydrolysiert unter dem Einfluß des bei der Knallgasreaktion entstehenden Wassers zu Siliciumdioxid und Salzsäure. Das Siliciumdioxid tritt nach dem Verlassen der Flamme in eine sogenannte Koagulationszone ein, in der die AEROSIL® -Primärteilchen und -Primäraggregate agglomerieren. Das in diesem Stadium als eine Art Aerosol vorliegende Produkt wird in Zyklonen von den gasförmigen Begleitsubstanzen getrennt und anschließend mit feuchter Heißluft nachbehandelt. Durch dieses Verfahren läßt sich der Rest-Salzsäuregehalt unter 0,025 Gew.-% senken. Da das AEROSIL® am Ende dieses Prozesses mit einer Schüttdichte von nur ca. 15 g/l anfällt, wird eine Vakuumverdichtung, mit der sich Stampfdichten von ca. 50 g/l und mehr einstellen lassen, angeschlossen.

Die Teilchengrößen der auf diese Weise gewonnenen Produkte können mit Hilfe der Reaktionsbedingungen, wie zum Beispiel die Flammentemperatur, der Wasserstoff- oder Sauerstoffanteil, die Siliciumtetrachloridmenge, die Verweilzeit in der Flamme oder die Länge der Koagulationsstrecke, variiert werden.

Die BET-Oberfläche wird gemäß DIN 66 131 mit Stickstoff bestimmt. Das Porenvolumen wird rechnerisch aus der Summe von Mikro-, Meso- und Makroporenvolumen bestimmt.

Die Bestimmung der Mikro- und Mesoporen erfolgt durch Aufnahme einer N₂-Isotherme und deren Auswertung nach BET, de Boer und Barret, Joyner, Halenda.

Die Bestimmung der Makroporen erfolgt durch das Hg-Einpreßverfahren.

Die Erfindung ist durch die nachstehenden Beispiele erläutert.

### Beispiel 1

| | |
|---|---|
| 85 Gew.-% | Aerosil® 200 |
| 5 Gew.-% | Methylhydroxyethylcellulose |
| 5 Gew.-% | Wachs |
| 5 Gew.-% | Polyethylenglycol |

werden unter Zusatz von Wasser, welches mit einer wäßrigen, ammoniakalischen Lösung (15 ml einer 32%igen Lösung für eine 2 kg Ansatz) schwach alkalisch gestellt wird, in einem Kneter kompaktiert. Die Knetmasse wird in einem Einschneckenextruder zu Hohlzylinder-Strangpreßlingen in Form von sogenannten Wagenrädern mit fünf internen verstärkenden Speichen oder Stegen ausgehend von der inneren Wand des Hohlzylinders zum Zentrum des Formkörpers geformt und mit einer Schneidvorrichtung auf die gewünschte Länge von 3,5 bis 5,5 mm geschnitten. Die Formkörper werden in einem Bandtrockner bei 90°C getrocknet und anschließend 6 Stunden bei 900°C calcinert.

Die erhaltenen Formkörper weisen folgende physikalisch-chemischen Kenndaten auf:

| | |
|---|---|
| Formkörperabmessungen: Aussendurchmesser (mm) | 7,5 ± 0,5 |
| Höhe(mm) | 4,5 ± 1 |
| Wandstärke: | 1,3 ± 0,05 |
| Stegbreite: | 1,3 ± 0,05 |
| BET-Oberfläche (m²/g) | 79 |
| Porenvolumen (ml/g) | 0.69 |
| Schüttdichte (g/l) | 398 |
| SiO₂-Gehalt (Gew.-%) | 99,9 |
| Verhältnis Höhe/Durchmesser | 0,6 |

### Beispiel 2

| | |
|---|---|
| 85 Gew.-% | Aerosil® 200 |
| 5 Gew.-% | Methylhydroxyethylcellulose |
| 5 Gew.-% | Wachs |
| 5 Gew.-% | Polyethylenglycol |

werden unter Zusatz von Wasser, welches mit einer wäßrigen, ammoniakalischen Lösung (15 ml einer 32%igen Lösung für eine 2 kg Ansatz) schwach alkalisch gestellt wird, in einem Kneter kompaktiert. Die Knetmasse wird in einem Einschneckenextruder zu Hohlzylinder-Strangpreßlingen in Form von sogenannten Wagenrädern mit fünf internen verstärkenden Speichen oder Stegen ausgehend von der inneren Wand des Hohlzylinders zum Zentrum des Formkörpers geformt und mit einer Schneidvorrichtung auf die gewünschte Länge von 5,5 bis 6,5 mm geschnitten. Die Formkörper werden in einem Bandtrockner bei 90°C getrocknet und anschließend 6 Stunden bei 850°C calcinert.

Die erhaltenen Formkörper weisen folgende physikalischchemischen Kenndaten auf:

| | |
|---|---|
| Formkorperabmessungen: Aussendurchmesser (mm) | 6,0 ± 0,2 |
| Höhe (mm) | 6,0 ± 0,5 |
| Wandstärke: | 0,95 ± 0,05 |
| Stegbreite: | 0,95 ± 0,05 |
| BET-Oberfläche (m²/g) | 148 |
| Porenvolumen (ml/g) | 0,75 |
| Schüttdichte (g/l) | 390 |
| SiO₂-Gehalt (Gew.-%) | 99,9 |
| Verhältnis Höhe/Durchmesser | 1,0 |

### Beispiel 3

| | |
|---|---|
| 85 Gew.-% | Aerosil® 200 |
| 5 Gew.-% | Methylhydroxyethylcellulose |
| 5 Gew.-% | Wachs |
| 5 Gew.-% | Polyethylenglycol |

werden unter Zusatz von Wasser, welches mit einer wäßrigen, ammoniakalischen Lösung (15 ml einer 32%igen Lösung für einen 2 kg Ansatz) schwach alkalisch gestellt wird, in einem Kneter kompaktiert. Die Knetmasse wird in einem Einschneckenextruder zu Hohlzylinder-Strangpreßlingen in Form von sogenannten Wagenrädern mit fünf internen verstärkenden Speichen oder Stegen ausgehend von der inneren Wand des Hohlzylinders zum Zentrum des Formkörpers geformt und mit einer Schneidvorrichtung auf die gewünschte Länge von 3,5 bis 5,5 mm geschnitten. Die Formkörper werden in einem Bandtrockner bei 90°C getrocknet und anschließend 6 Stunden bei 800°C calcinert.

Die erhaltenen Formkörper weisen folgende physikalisch-chemischen Kenndaten auf:

| | |
|---|---|
| Formkörperabmessungen: Aussendurchmesser (mm) | 7,5 ± 0,5 |
| Höhe(mm) | 4,5 ± 1 |
| Wandstärke: | 1,3 ± 0,05 |
| Stegbreite: | 1,3 ± 0,05 |
| BET-Oberfläche (m²/g) | 170 |
| Porenvolumen (ml/g) | 0.9 |
| Schüttdichte (g/l) | 360 |
| SiO₂-Gehalt (Gew.-%) | 99,9 |
| Verhältnis Höhe /Durchmesser | 0,6 |

### Beispiel 4

| | |
|---|---|
| 85 Gew.-% | Aerosil® 200 |
| 5 Gew.-% | Methylhydroxyethylcellulose |
| 5 Gew.-% | Wachs |
| 5 Gew.-% | Polyethylenglycol |

werden unter Zusatz von Wasser, welches mit einer wäßrigen, ammoniakalischen Lösung (15 ml einer 32%igen Lösung für eine 2 kg Ansatz) schwach alkalisch gestellt wird, in einem Kneter kompaktiert. Die Knetmasse wird in einem Einschneckenextruder zu Hohlzylinder-Strangpreßlingen in Form von sogenannten Wagenrädern mit fünf internen verstärkenden Speichen oder Stegen ausgehend von der inneren Wand des Hohlzylinders zum Zentrum des Formkörpers geformt und mit einer Schneidvorrichtung auf die gewünschte Länge von 5,5 bis 6,5 mm geschnitten. Die Formkörper werden in einem Bandtrockner bei 90°C getrocknet und anschließend 6 Stunden bei 800°C calcinert.

Die erhaltenen Formkörper weisen folgende physikalisch-chemischen Kenndaten auf:

| | |
|---|---|
| Formkörperabmessungen: Aussendurchmesser (mm) | 6,0 ± 0,2 |
| Höhe (mm) | 6,0 ± 0,5 |
| Wandstärke: | 0,95 ± 0,05 |
| Stegbreite: | 0,95 ± 0,05 |
| BET-Oberfläche (m²/g) | 170 |
| Porenvolumen (ml/g) | 0,9 |
| Schüttdichte (g/l) | 350 |
| SiO₂-Gehalt (Gew.-%) | 99,9 |
| Verhältnis Höhe/Durchmesser | 1,0 |

### Beispiel 5

| | |
|---|---|
| 85 Gew.-% | Aerosil® 200 |
| 5 Gew.-% | Methylhydroxyethylcellulose |
| 5 Gew.-% | Wachs |
| 5 Gew.-% | Polyethylenglycol |

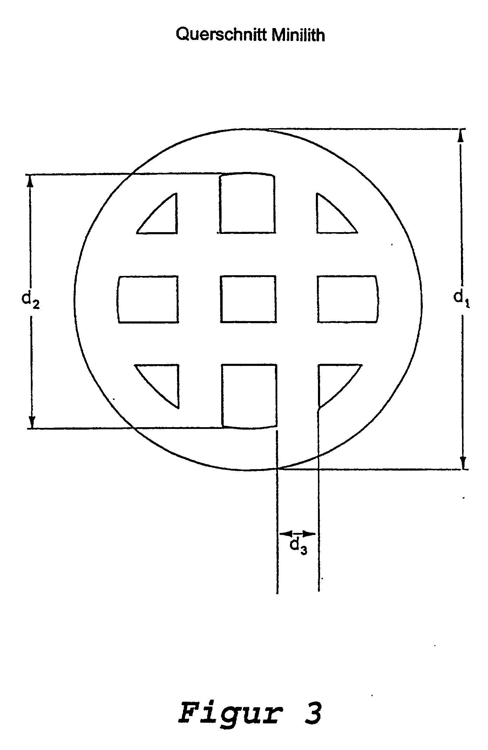

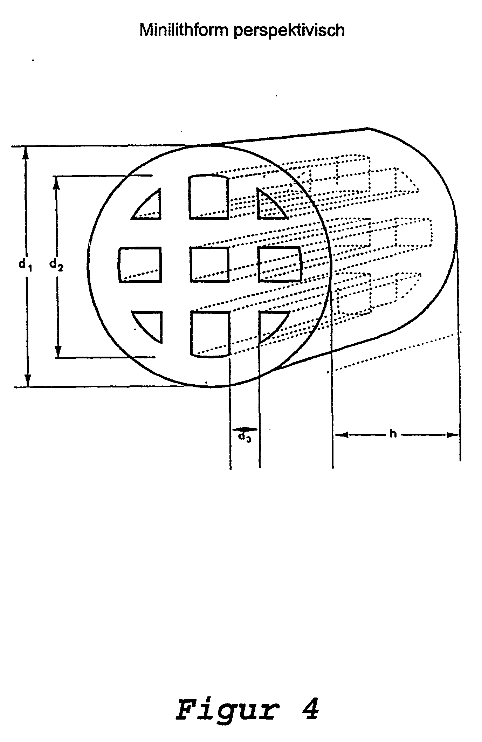

werden unter Zusatz von Wasser, welches mit einer wäßrigen, ammoniakalischen Lösung (15 ml einer 32%igen Lösung für eine 2 kg Ansatz) schwach alkalisch gestellt wird, in einem Kneter kompaktiert. Die Knetmasse wird in einem Einschneckenextruder zu Hohlzylinder-Strangpreßlingen in Form von sogenannten Minilithen gemäß Figur 3 bzw. Figur 4 mit neun Durchtrittskanälen geformt und mit einer Schneidvorrichtung auf die gewünschte Länge von 4 bis 5 mm geschnitten. Die Formkörper werden in einem Bandtrockner bei 90°C getrocknet und anschließend bei 800°C calcinert.

Die erhaltenen Formkörper weisen folgende physikalischchemischen Kenndaten auf:

| | |
|---|---|
| Formkörperabmessungen: Aussendurchmesser (mm) | 5,8 ± 0,2 |
| Höhe(mm) | 4,5 ± 0,5 |
| Wandstärke: | 0,8 ± 0,05 |
| Stegbreite: | 0,8 ± 0,05 |
| BET-Oberfläche (m²/g) | 170 |
| Porenvolumen (ml/g) | 0,9 |
| Schüttdichte (g/l) | 350 |
| SiO₂-Gehalt (Gew.-%) | 99,9 |
| Verhältnis Höhe/Durchmesser | 0,78 |

### Vergleichsbeispiel 1

Es wird ein Palladium-Gold-Kaliumacetat-Katalysator nach Beispiel 11 der EP 0 807 615 A1 hergestellt. Als Katalysatorträger wird ein Formkörper nach Beispiel 5 der EP 0 807 615 A1, jedoch mit den Abmessungen 8 x 5 x 3 mm (Aussendurchmesser x Höhe x Innendurchmesser) und Facettenkanten eingesetzt.

Die Konzentration der Imprägnierlösungen wird so gewählt, daß der fertige Katalysator eine Konzentration von 0,55 Gew.-% Palladium, 0,25 Gew.-% Gold und 5,0 Gew.-% Kaliumacetat enthält.

In einem ersten Schritt wird der Träger zunächst mit einer basischen Lösung aus Natriumhydroxid in Wasser imprägniert. Das Volumen der wäßrigen NaOH-Lösung entspricht 50 Prozent der Wasseraufnahme des trockenen Trägers. Nach der Imprägnierung mit Natriumhydroxid wird der Träger unmittelbar ohne Zwischentrocknung mit einer wäßrigen Edelmetalllösung aus Natriumpalladiumchlorid und Tetrachlorogoldsäure imprägniert, deren Volumen ebenfalls 50 Prozent der Wasseraufnahmekapazität des trockenen Trägers entspricht.

Nach einer Wartezeit von 1,5 Stunden, während der die Edelmetallverbindungen hydrolysieren, werden die Trägerteilchen chloridfrei gewaschen. Die Trägerteilchen werden getrocknet und bei 450°C in der Gasphase mit Formiergas (95 Vol.-% N₂, 5 Vol.-% H₂) reduziert. Danach wird der Katalysator mit einer wäßrigen Kaliumacetat-Lösung imprägniert und erneut getrocknet. Die Trocknung wird in der Gasphase mit Stickstoff durchgeführt.

Die Konzentration der basischen Lösung an Natriumhydroxid ist so bemessen, daß sich auf den Trägerteilchen eine edelmetallhaltige Schale von < 1,0 mm ausbildet.

### Beispiel 6

Es wird ein Palladium-Gold-Kaliumacetat-Katalysator wie in Vergleichsbeispiel 1 beschrieben auf dem erfindungsgemäßen Formkörper nach Beispiel 1 hergestellt.

### Beispiel 7

Es wird ein Palladium-Gold-Kaliumacetat-Katalysator wie in Vergleichsbeispiel 1 beschrieben auf dem erfindungsgemäßen Formkörper nach Beispiel 2 hergestellt.

### Anwendungsbeispiel 1

Aktivität und Selektivität der Katalysatoren aus dem Vergleichsbeispiel 1 und den Beispielen 6 und 7 werden während einer Prüfung über die Dauer von bis zu 24 Stunden gemessen.

Die Katalysatoren werden in einem mit Öl beheizten Strömungsrohrreaktor (Reaktorlänge 710 mm, Innendurchmesser 23,7 mm) bei Normaldruck und einer Raumgeschwindigkeit (GHSV) von 400 h⁻¹ mit der folgenden Gaszusammensetzung geprüft: 75 Vol.-% Ethen, 16,6 Vol.-% Essigsäure, 8,3 Vol.-% Sauerstoff. Die Katalysatoren werden im Temperaturbereich von 120 bis 165°C, gemessen im Katalysatorbett, untersucht.

Die Reaktionsprodukte werden im Ausgang des Reaktors mittels on-line Gaschromatographie analysiert. Als Maß für die Katalysatoraktivität wird die Raum-Zeit-Ausbeute des Katalysators in Gramm Vinylacetat-Monomer pro Stunde und Kilogramm Katalysator (g VAM/(h x kg_{Kat.}) bestimmt.

Kohlendioxid, das insbesondere durch die Verbrennung von Ethen gebildet wird, wird ebenfalls bestimmt und zur Beurteilung der Katalysatorselektivität herangezogen.

In Tabelle 1 sind die Untersuchungsergebnisse an den Katalysatoren aus dem Vergleichsbeispiel 1 und den Beispielen 6 und 7 dargestellt. Die Katalysatoraktivität und die Katalysatorselektivität des Katalysators nach Vergleichsbeispiel 1 werden jeweils als 100 Prozent gesetzt.

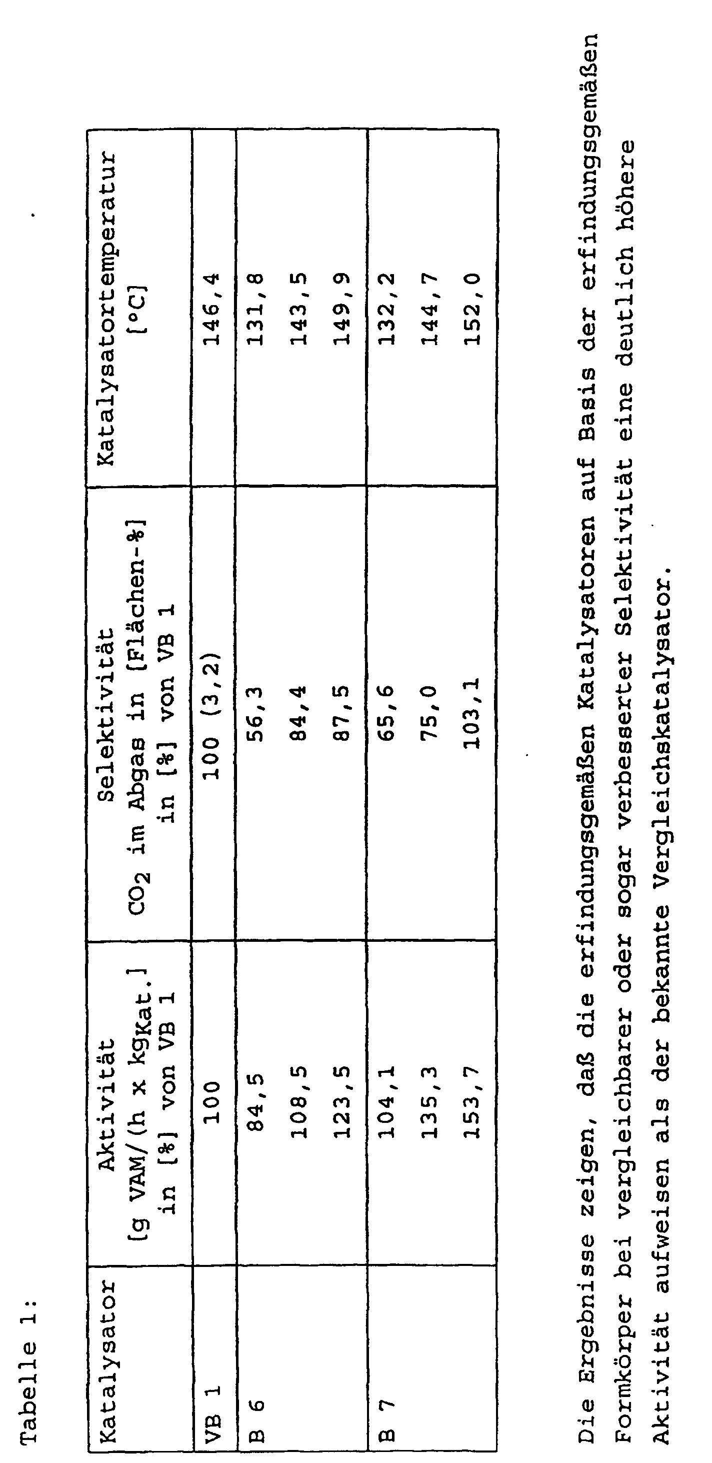

## Patentansprüche

1. Formkörper auf Basis von Siliciumdioxid, **dadurch gekennzeichnet, daß** die Trägergeometrie durch eine Hohlzylinder-Konfiguration mit internen verstärkenden Stegen oder Speichen ausgehend von der inneren Wand des Hohlzylinders zum Zentrum des Formkörpers besteht oder durch eine Vielzahl von Durchtrittskanälen charakterisiert ist, wobei der Formkörper einen Aussen-Durchmesser von 1 bis zu 25 mm und ein Verhältnis von Höhe zu Durchmesser 0,2 bis 5 aufweist und als Siliciumdioxid ein pyrogen hergestelltes Siliciumdioxid eingesetzt wird.

2. Formkörper nach Anspruch 1, **dadurch gekennzeichnet, daß** der Formkörper eine Wanddicke im Bereich des 0,05 bis 0,3 fachen des Durchmessers und eine Steg- bzw. Speichendicke im Bereich des 0,05 bis 0,3 fachen des Durchmessers aufweist.

3. Formkörper nach Anspruch 1, **dadurch gekennzeichnet, daß** die Anzahl der internen verstärkenden Stege oder Speichen oder Durchtrittskanäle mindestens 3 beträgt.

4. Verfahren zur Herstellung der Formkörper auf Basis von Siliciumdioxid gemäß Anspruch 1, **dadurch gekennzeichnet, daß** man ein pyrogen hergestelltes Siliciumdioxid einem Knet- und Verformungsverfahren unterzieht, extrudiert, die Extrudate gegebenenfalls mittels einer Schneidvorrichtung auf die gewünschte Länge schneidet, bei einer Temperatur von 20 bis zu 150°C trocknet und während eines Zeitraums von 0,5 bis zu 10 Stunden bei einer Temperatur von 400 bis 1200°C tempert.

5. Verfahren zur Herstellung der Formkörper auf Basis von Siliciumdioxid gemäß Anspruch 1, **dadurch gekennzeichnet, daß** man pyrogen hergestelltes Siliciumdioxid mit Methylhydroxyethylcellulose, Wachs und/oder Polyethylenglycol unter Zusatz von Wasser sowie gegebenenfalls unter Zugabe einer wäßrigen, ammoniakalischen Lösung homogenisiert, einem Knet- und Verformungsverfahren unterzieht, und/oder extrudiert, die Formkörper gegebenenfalls mittels einer Schneidvorrichtung auf die gewünschte Länge schneidet, bei einer Temperatur von 10 bis zu 150°C trocknet und während eines Zeitraums von 30 Minuten bis zu 10 Stunden bei einer Temperatur von 400 bis 1200°C tempert.

6. Verwendung der Formkörper nach Anspruch 1 als Katalysator oder Katalysatorträger.

7. Trägerkatalysator für die Herstellung von Vinylacetatmonomer (VAM), welcher auf einem Träger (Formkörper) als katalytisch aktive Komponenten Palladium und/oder dessen Verbindungen und Alkaliverbindungen, sowie zusätzlich Gold und/oder dessen Verbindungen (System Pd/Alkali/Au) oder Cadmium und/oder dessen Verbindungen (System Pd/Alkali/Cd) oder Barium und/oder dessen Verbindungen (System Pd/Alkali/Ba) oder Palladium, Alkaliverbindungen und Mischungen aus Gold und/oder Cadmium und/oder Barium enthält, **dadurch gekennzeichnet, daß** der Träger ein Formkörper gemäß Anspruch 1 ist.

8. Verfahren zur Herstellung des Trägerkatalysators nach Anspruch 7 für die Produktion von Vinylacetatmonomer durch Tränken, Aufsprühen, Aufdampfen, Tauchen oder Ausfällen der Pd-, Au-, Cd-, Ba-Metallverbindungen, gegebenenfalls Reduzieren der auf dem Träger aufgebrachten reduzierbaren Metallverbindungen, gegebenenfalls Waschen zur Entfernung gegebenenfalls vorhandener Chloridanteile, Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln, in geeigneter Reihenfolge, **dadurch gekennzeichnet, daß** der Träger ein Formkörper gemäß Anspruch 1 ist.

9. Verfahren zur Herstellung des Trägerkatalysators nach Anspruch 7 für die Produktion von Vinylacetatmonomer durch Imprägnieren des Trägers mit einer basischen Lösung und einer Gold- und Palladiumsalze enthaltenden Lösung, wobei die Imprägnierung gleichzeitig oder nacheinander, mit oder ohne Zwischentrocknung erfolgt, gegebenenfalls Waschen des Trägers zur Entfernung gegebenenfalls vorhandener Chloridanteile und Reduzieren der auf dem Träger ausgefällten unlöslichen Verbindungen vor oder nach dem Waschen, Trocknen der so erhaltenen Katalysatorvorstufe, und Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln, **dadurch gekennzeichnet, daß** der Träger ein Formkörper gemäß Anspruch 1 ist.

10. Verwendung des Trägerkatalysators gemäß Anspruch 7 zur Herstellung ungesättigter Ester aus Olefinen, organischen Säuren und Sauerstoff in der Gasphase.

## Claims

1. Moulded body based on silicon dioxide, **characterised in that** the support geometry consists of a hollow cylinder configuration with internal reinforcing stays or spokes extending from the inner wall of the hollow cylinder to the centre of the moulded body, or is **characterised by** a large number of penetration channels, the moulded body having an external diameter of 1 to 25 mm and a ratio of height to diameter of 0.2 to 5, and a pyrogenically-produced silicon dioxide is used as the silicon dioxide.

2. Moulded body according to claim 1, **characterised in that** the moulded body has a wall thickness in the range 0.05 to 0.3 of the diameter and a stay or spoke thickness in the range 0.05 to 0.3 of the diameter.

3. Moulded body according to claim 1, **characterised in that** the number of internal reinforcing stays or spokes or penetration channels is at least 3.

4. Process for the production of moulded bodies based on silicon dioxide according to claim 1, **characterised in that** a pyrogenically-produced silicon dioxide is subjected to a kneading and forming process, extruded, the extrudate optionally cut to the desired length using a cutting device, dried at a temperature of 20 to 150°C and tempered for a period of 0.5 to 10 hours at a temperature of 400 to 1200°C.

5. Process for the production of moulded bodies based on silicon dioxide according to claim 1, **characterised in that** pyrogenically-produced silicon dioxide is homogenised with methyl hydroxyethyl cellulose, wax and/or polyethylene glycol adding water and optionally adding an aqueous ammoniac solution, subjected to a kneading and forming process and/or extruded, the moulded bodies optionally cut to the desired length using a cutting device, dried at a temperature of 10 to 150°C and tempered for a period of 30 minutes to 10 hours at a temperature of 400 to 1200°C.

6. Use of the moulded bodies according to claim 1 as catalysts or catalyst supports.

7. Supported catalyst for the production of vinyl acetate monomer (VAM), which contains, on a support (moulded body), as catalytically active components palladium and/or compounds and alkali compounds thereof and additionally gold and/or compounds thereof (Pd/Alkali/Au system) or cadmium and/or compounds thereof (Pd/Alkali/Cd system) or barium and/or compounds thereof (Pd/Alkali/Ba system), or palladium, alkali compounds and mixtures of gold and/or cadmium and/or barium, **characterised in that** the support is a moulded body according to claim 1.

8. Process for the production of the supported catalyst according to claim 7 for the production of vinyl acetate monomer, by impregnating, spray coating, evaporation coating, dipping or depositing the Pd-, Au-, Cd-, Ba-metal compounds, optionally reducing the reducible metal compounds applied to the support, optionally washing to remove any chloride particles, impregnating with alkali acetates or alkali compounds which, under the reaction conditions for the production of vinyl acetate monomer, are wholly or partly converted into alkali acetates, in a suitable order, **characterised in that** the support is a moulded body according to claim 1.

9. Process for the production of the supported catalyst according to claim 7 for the production of vinyl acetate monomer, by impregnating the support with a basic solution and a solution containing gold and palladium salts, impregnation taking place simultaneously or successively, with or without intermediate drying, optionally washing the support to remove any chloride particles and reducing the insoluble compounds deposited on the support before or after washing, drying the catalyst precursor thus obtained and impregnating it with alkali acetates or alkali compounds which, under the reaction conditions for production of vinyl acetate monomer, are wholly or partly converted into alkali acetates, **characterised in that** the support is a moulded body according to claim 1.

10. Use of the supported catalyst according to claim 7 for the production of unsaturated esters from olefins, organic acids and oxygen in the gas phase.

## Revendications

1. Corps moulés à base à base de silice,
**caractérisés en ce que**
la géométrie du support consiste en une configuration en cylindre creux avec des cloisons ou rayons internes de renfort partant de la paroi interne du cylindre creux vers le centre des corps moulés, ou **caractérisé par** une multiplicité de canaux de passage, le corps moulé présentant un diamètre externe de 1 à 25 mm et un rapport de la hauteur au diamètre de 0,2 à 5, et on utilise comme silice une silice préparée par pyrogénation.

2. Corps moulé selon la revendication 1,
**caractérisé en ce que**
le corps moulé présente une épaisseur de paroi dans la plage représentant de 0,05 à 0,3 fois le diamètre, et une épaisseur de rayon ou de cloison dans la plage représentant de 0,05 à 0,3 fois le diamètre.

3. Corps moulé selon la revendication 1,
**caractérisé en ce que**
le nombre des cloisons ou rayons internes de renfort ou des canaux de passage est égal au moins à 3.

4. Procédé pour la fabrication des corps moulés à base de silice selon la revendication 1,
**caractérisé en ce qu'**
on soumet de la silice préparée par pyrogénation à un procédé de malaxage et de mise en forme, on l'extrude, les extrudats sont éventuellement coupés à la longueur désirée au moyen d'un dispositif de coupe, séchés à une température de 20 à 150°C, et traités thermiquement à une température de 400 à 1 200°C, pendant une durée de 0,5 à 10 heures.

5. Procédé pour la fabrication des corps moulés à base de silice selon la revendication 1,
**caractérisé en ce qu'**
on homogénéise de la silice, préparée par pyrogénation, avec de la méthylhydroxyéthylcellulose, une cire et/ou du polyéthylèneglycol, avec addition d'eau, ainsi qu'éventuellement avec addition d'une solution aqueuse ammoniacale, on la soumet à une processus de malaxage et de mise en forme, et/ou on l'extrude, les corps moulés sont éventuellement coupés à la longueur désirée au moyen d'un dispositif de coupe, séchés à une température de 10 à 150°C et soumis à un traitement thermique à une température de 400 à 1 200°C pendant une durée de 30 minutes à 10 heures.

6. Utilisation des corps moulés selon la revendication 1, en tant que catalyseur ou support de catalyseur.

7. Catalyseur sur support pour la production du monomère acétate de vinyle (VAM), qui contient sur un support (corps moulé), en tant que composant catalytiquement actif, du palladium et/ou des composés de celui-ci et des composés alcalins, ainsi qu'en outre de l'or et/ou des composés de celui-ci (système Pd/alcali/Au) ou du cadmium et/ou des composés de celui-ci (système Pd/alcali/Cd) ou du baryum et/ou des composés de celui-ci (système Pd/alcali/Ba) ou du palladium, des composés alcalins et des mélanges d'or et/ou de cadmium et/ou baryum,
**caractérisé en ce que**
le support est un corps moulé selon la revendication 1.

8. Procédé pour la fabrication du catalyseur sur support selon la revendication 7, pour la production de monomère acétate de vinyle par imprégnation, pulvérisation, dépôt par vaporisation, immersion ou précipitation des composés métalliques à base de Pd, Au, Cd, Ba, éventuellement réduction des composés métalliques réductibles appliqués sur le support, éventuellement lavage pour l'élimination d'éventuellement présentes fractions contenant des chlorures, imprégnation avec des acétates alcalins ou des composés alcalins qui se convertissent en partie ou en totalité en acétates de métaux alcalins dans les conditions réactionnelles régnant dans la production du monomère acétate de vinyle, en un ordre approprié,
**caractérisé en ce que**
le support est un corps moulé selon la revendication 1.

9. Procédé pour la fabrication du catalyseur sur support selon la revendication 7, pour la production de monomère acétate de vinyle par imprégnation du support avec une solution basique et une solution contenant des sels d'or et de palladium, l'imprégnation s'effectuant simultanément ou successivement, avec ou sans séchage intermédiaire, éventuellement lavage du support pour l'élimination d'éventuellement présentes fractions contenant des chlorures et réduction des composés insolubles précipités sur le support, avant ou après le lavage, séchage du précurseur de catalyseur ainsi obtenu, et imprégnation avec des acétates de métaux alcalins ou des composés alcalins qui se convertissent en partie ou en totalité en acétates alcalins dans les conditions réactionnelles régnant dans la production du monomere acétate de vinyle,
**caractérisé en ce que**
le support est un corps moulé selon la revendication 1.

10. Utilisation du catalyseur sur support selon la revendication 7, pour la production d'esters insaturés à partir d'oléfines, d'acides organiques et d'oxygène en phase gazeuse.
